Europäisches Patentamt

European Patent Office

Office européen des brevets

(11) Veröffentlichungsnummer: **0 075 203**
**B1**

(12) **EUROPÄISCHE PATENTSCHRIFT**

(45) Veröffentlichungstag der Patentschrift:
02.05.85

(51) Int. Cl.⁴: **C 07 C 1/20, C 07 C 11/02**

(21) Anmeldenummer: **82108288.0**

(22) Anmeldetag: **09.09.82**

(54) **Verfahren zur Herstellung von Olefinen aus Methanol/Dimethylether.**

(30) Priorität: **17.09.81 DE 3136984**

(43) Veröffentlichungstag der Anmeldung:
**30.03.83 Patentblatt 83/13**

(45) Bekanntmachung des Hinweises auf die Patenterteilung:
**02.05.85 Patentblatt 85/18**

(84) Benannte Vertragsstaaten:
**BE DE FR GB IT NL**

(56) Entgegenhaltungen:
**EP - A - 0 011 900
EP - A - 0 017 027
WO - A - 82/01866**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Hoelderich, Wolfgang, Dr., Mannheimer Strasse 18 c, D-6710 Frankenthal (DE)**
Erfinder: **Mross, Wolf Dieter, Dr.,
Anselm-Feuerbach-Strasse 21, D-6710 Frankenthal (DE)**
Erfinder: **Schwarzmann, Matthias, Dr.,
Carl-Bosch-Strasse 54, D-6703 Limburgerhof (DE)**

## Beschreibung

In neuerer Zeit gewinnen Bemühungen, Olefine aus Methanol herzustellen zunehmendes Interesse. Methanol kann man aus Kohle, durch Kohlevergasung und aus Synthesegas, mit Hilfe bewährter Technologien herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff weiter beibehalten werden. In den vergangenen Jahren sind daher Verfahren entwickelt worden, die die Herstellung von Olefinen aus Methanol und/oder Dimethylether zum Gegenstand haben. Ein solches Verfahren ist beispielsweise in der DE-OS Nr. 2615150 beschrieben. Die Umsetzung kann durch verschiedene Massnahmen, z. B. durch Verkürzung der Verweilzeit, optimiert werden. Weitere die Olefinbildung begünstigende Parameter sind insbesondere die Verdünnung von Methanol bzw. Dimethylether mit Inertgasen bzw. Wasserdampf oder die Verdünnung des Katalysators mit Bindemitteln.

Es wurde nun gefunden, dass man $C_2$- bis $C_4$-Olefine in hoher Ausbeute aus Methanol und/oder Dimethylether durch Umsetzung in Gegenwart von Zeolithkatalysatoren bei erhöhter Temperatur erhält, wenn man die Umsetzung unter Zugabe von Elektronendonatoren in Mengen von 1 bis 300 ppm vornimmt.

Als Elektronendonatoren, die den Ausgangsstoffen zugesetzt werden, sind im Sinne der Erfindung z. B. die nachfolgend aufgeführten Stoffklassen zu verstehen. Allgemein sind als Elektronendonatoren Stoffe geeignet, die Lewis-Basizität besitzen.

Der besondere technische Effekt des Zusatzes von Elektronendonatoren zu den Ausgangsstoffen vor der Umsetzung an Zeolithkatalysatoren liegt in deren selektivitätssteigerndem und laufzeitverbesserndem Einfluss. Die Selektivität der Umsetzung wird in Richtung der Bildung der Olefine, insbesondere Ethylen, Propylen und Butenen gelenkt. Der Anteil der $C_5{}^+$-Kohlenwasserstoffe im Reaktionsprodukt geht zurück.

Eine bevorzugte Ausführungsform des erfindungsgemässen Verfahrens besteht darin, dem Methanol 1 bis 10 ppm eines Elektronendonators zuzusetzen und die Gemische bei einem Druck zwischen Normaldruck und 30 bar und bei Temperaturen zwischen 300 und 700° C, bevorzugt zwischen 400 und 550° C an einem Borosilikatzeolithen umzusetzen.

Die Ausgangsstoffe z. B. das Methanol kann einen Wassergehalt bis zu 90 Gew.-% haben. Bevorzugt wird Rohmethanol, wie es aus der Methanolsynthese kommt, mit etwa 20 Gew.-% Wasser eingesetzt. Dem Methanol können auch noch andere niedere Alkohole beigemischt sein. Die Belastung des Katalysators, ausgedrückt in WHSV = $h^{-1}$ — g Methanol und/oder Dimethylether pro Gramm Katalysator und Stunde —, wird vorteilhafterweise so gewählt, dass diese Einsatzstoffe möglichst quantitativ umgesetzt werden, damit keine Ab-

trenn- und Rückführprobleme entstehen. Im allgemeinen wird daher WHSV im Bereich von 0,5 bis 50, vorzugsweise von 2 bis 15 $h^{-1}$, liegen. Es ist ein besonderer Vorteil der Erfindung, dass man die Umsetzung von Rohmethanol bzw. Dimethylether in $C_2$- bis $C_4$-Olefine ohne Verdünnungsmittel ausführen kann.

Beispiele für Elektronendonatoren, die den Ausgangsstoffen zugesetzt werden können, sind nachfolgende Verbindungen:

1. Substituierte Kohlenwasserstoffe

$X_n(CR_1R_2)_qY_m$

$X_nCY_mZ_l$

$X_nY_mZ_lC-(R)_{4-m-n-l}$

X = F, Cl, Br, J, H, O, S, N

Y = F, Cl, Br, J, H, O, S, N

Z = F, Cl, Br, J, H, O, S, N

$R = R^1 = R^2$ = Wasserstoff,

Alkylgruppe wie $CH_3-$, $C_2H_5-$,

Alkoxylgruppe wie $CH_3O-$, $C_2H_5O-$,

Kethylgruppe wie $-CO-CH_3$, $-CO-C_2H_5$,

Säuregruppe wie $-COOH$,

Estergruppe wie $-COOCH_3$, $-COOC_2H_5$,

Aminogruppe wie $-N(CH_3)_2$, $-N(C_2H_5)_2$,

l = 1 bis 4

m = 1 bis 4

n = 1 bis 4

q = 1 bis 10

2. Andere geeignete Kohlenwasserstoffe sind organische Säuren und Amine.

3. Substituierte Silane

$X_n(SiR_2)_qY_m$

$X_nSiY_mZ_l$

$X_nY_mZ_lSiR_{4-n-m-l}$

X = F, Cl, Br, J, H

Y = F, Cl, Br, J, H

Z = F, Cl, Br, J, H

R = Wasserstoff,

Alkylgruppe wie $CH_3-$, $C_2H_5-$,

Alkoxylgruppe wie $CH_3O-$, $C_2H_5O-$,

Kethylgruppe wie $-CO-CH_3$, $-CO-C_2H_5$, $-CO-CH_3$,

Säuregruppe wie $-COOH$,

Estergruppe wie $-COOCH_3$,

Aminogruppe wie $-N(CH_3)_2$, $-N(C_2H_5)_2$

l = 1 bis 4

m = 1 bis 4

n = 1 bis 4

q = 1 bis 4

4. Andere geeignete siliciumhaltige Verbindungen sind Siloxane wie Disiloxane, Trisiloxane, Silylamine wie Trimethylsilylamin, Silylphosphine wie Trimethylsilylphosphin und Kieselsäuren und deren Ester.

5. Substituierte Schwefelverbindungen

$X_nSY_m$

$X_nY_mSR_{6-m-n}$

X = F, Cl, Br, J, H, O

Y = F, Cl, Br, J, H, O

R = z. B. Wasserstoff,

Alkylgruppe,
Alkoxylgruppe,

$m = 1$ bis 6
$n = 1$ bis 6

6. Andere geeignete Schwefelverbindungen sind die Säuren des Schwefels und deren Ester.

7. Substituierte P-, As-, Sb-Verbindungen

$$X_m Y_n Z_l PR_{5-m-n-l}$$
$$X_m Y_n Z_l AsR_{5-m-n-l}$$
$$X_m Y_n Z_l SbR_{5-m-n-l}$$

$X = F, Cl, Br, J, H, O, S$
$Y = F, Cl, Br, J, H, O, S$
$Z = F, Cl, Br, J, H, O, S$
$R =$ Wasserstoff,

Alkylgruppe,
Alkoxylgruppe,
Aminogruppe wie $-N(CH_3)_2$,
Phosphinogruppe wie $-P(CH_3)_2$

$l = 1$ bis 5
$m = 1$ bis 5
$n = 1$ bis 5

8. Andere geeignete Phosphor-, Arsen- und Antimonverbindungen sind die Säuren dieser Elemente und deren Ester, ebenso Alkyl-, Aryl- und Alkoxylverbindungen dieser Elemente.

Die Durchführung des erfindungsgemässen Verfahrens wird anhand des nachstehenden Beispiels näher erläutert.

Der Borzeolith kann wie folgt hergestellt werden:

Der Borzeolith wird in einer hydrothermalen Synthese hergestellt aus 66,1 g $SiO_2$, 30,8 g $H_3BO_3$ in 881 g einer wässerigen 1,3-Propandiaminlösung (Mischung 50:50) bei 170° C unter autogenem Druck in einem Rührautoklaven. Nach Abfiltrieren und Auswaschen wird das kristalline Produkt bei 160° C 24 h getrocknet und bei 500° C 24 h calciniert. Der gebildete Borosilikatzeolith setzt sich zusammen aus 91,2% $SiO_2$ und 4,74% $B_2O_3$. Nach Verstrangung des Zeoliths mit Beohmit im Verhältnis 60:40 erhält man den in den nachfolgenden Versuchen verwendeten Zeolithkatalysator.

*Beispiele 1 bis 3:*

In den Beispielen 1 bis 3, wobei Beispiel 1 ein Vergleichsbeispiel darstellt, wird die Umwandlung von Rohmethanol am oben beschriebenen Katalysator zu Olefinen beschrieben, und zwar mit und ohne Zusatz von Dichlorethan zum Einsatzstoff (Tabelle 1). Die Umsetzung wird bei 450° C und einer Belastung WHSV = 7,8 $h^{-1}$ durchgeführt. Der Methanolumsatz ist quantitativ.

*Tabelle 1*

*Einfluss des Dichlorethans*

| Beispiel | 1 | 2 | 3 |
|---|---|---|---|
| $ClCH_2-CH_2Cl$ | — | 1 ppm | 5 ppm |
| $CH_4$ | 2,0% | 2,5% | 2,6% |
| $C_2H_4$ | 7,6% | 9,3% | 9,0% |
| $C_2H_6$ | 0,2% | 0,2% | 0,3% |
| $C_3H_6$ | 32,5% | 37,2% | 36,4% |
| $C_3H_8$ | 2,1% | 2,8% | 2,7% |
| $C_4H_8$ | 22,5% | 26,5% | 28,8% |
| $C_4H_{10}$ | 5,2% | 6,5% | 5,3% |
| $C_5^+$ | 25 % | 13,8% | 13,5% |
| $\Sigma C$ | 97,1% | 98,8% | 98,6% |
| g $CH_3OH$/ g Katalysator | 320 g | 304 g | 390 g |

Die angegebenen Prozentgehalte geben die Ausbeuten an Kohlenwasserstoffen, bezogen auf eingesetztes $CH_2$, wieder.

In Tabelle 1 erkennt man, dass sich der Zusatz von Dichlorethan positiv auf die Selektivität der Methanolumwandlung zu den $C_2$-$C_4$-Olefinen auswirkt. Auch die Standzeit zwischen bis zur 1. Regenerierung des Katalysators, wird durch den Zusatz verbessert.

*Beispiele 4 bis 10:*

In den Beispielen 4 bis 10, wobei Beispiel 4 ein Vergleichsbeispiel darstellt, wird die Umwandlung von Rohmethanol, dem verschiedene Elektronendonatoren zugesetzt werden, bei 500° C und mit WHSV = $7,8^{-1}$ zu niederen Olefinen mitgeteilt (Tabelle 2). Das Methanol wird vollständig umgesetzt.

*Tabelle 2*

*Einfluss verschiedener Elektronendonatoren*

| Beispiel | 4 | 5 | 6 | 7 | 8 | 9 | 10 |
|---|---|---|---|---|---|---|---|
| Zusatz | — | $CCl_4$ | HCl | $CH_2Cl_2$ | $SOCl_2$ | ®Freon 12 | $(CH_3)_3SiF$ |
| $CH_4$ | 3,7% | 3,9% | 4,3% | 5,0% | 5,2% | 3,9% | 4,8% |
| $C_2H_4$ | 9,5% | 10,9% | 10,9% | 10,2% | 11,0% | 11,0% | 10,3% |
| $C_2H_6$ | 0,3% | 0,4% | 0,3% | 0,4% | 0,4% | 0,4% | 0,4% |
| $C_3H_6$ | 36,9% | 42,8% | 42,2% | 40,6% | 40,3% | 40,2% | 40,0% |
| $C_3H_8$ | 1,2% | 1,9% | 1,9% | 1,7% | 2,0% | 2,2% | 1,7% |
| $C_4H_8$ | 17,3% | 25,9% | 24,0% | 24,3% | 24,6% | 22,5% | 24,1% |
| $C_4H_{10}$ | 1,5% | 2,3% | 2,3% | 2,4% | 2,5% | 3,9% | 2,8% |
| $C_5^+$ | 29,0% | 8,0% | 9,4% | 11,6% | 10,7% | 11,9% | 10,7% |

® eingetragenes Warenzeichen

Die angegebenen Werte entsprechen den Ausbeuten an Kohlenwasserstoffen bezogen auf eingesetztes $CH_2$.

Auch aus Tabelle 2 ersieht man insbesondere an der Abnahme des $C_5^+$-Anteiles den selektivitätssteigernden Einfluss der Elektronendonatoren in Richtung der vermehrten Bildung von Ethylen, Propylen und Butenen.

**Patentansprüche**

1. Verfahren zur Herstellung von Olefinen aus Methanol und/oder Dimethylether durch Umsetzung in Gegenwart von Zeolithkatalysatoren bei erhöhter Temperatur, dadurch gekennzeichnet, dass man die Umsetzung unter Zugabe von Elektronendonatoren in Mengen von 1 bis 300 ppm vornimmt.

2. Verfahren nach Anspruch 1, dadurch gekennzeichnet, dass man die Umsetzung in Gegenwart von Borosilikatzeolithen vornimmt.

3. Verfahren nach den Ansprüchen 1 und 2, dadurch gekennzeichnet, dass man den Ausgangsstoffen als Elektronendonatoren substituierte Kohlenwasserstoffe zusetzt.

4. Verfahren nach Anspruch 3, dadurch gekennzeichnet, dass man den Ausgangsstoffen als Elektronendonatoren organische Säuren und Amine zusetzt.

5. Verfahren nach den Ansprüchen 1 und 2, dadurch gekennzeichnet, dass man den Ausgangsstoffen als Elektronendonatoren substituierte Silane zusetzt.

6. Verfahren nach den Ansprüchen 1 und 2, dadurch gekennzeichnet, dass man den Ausgangsstoffen als Elektronendonatoren geeignete siliciumhaltige Verbindungen, wie Siloxane, Disiloxane, Trisiloxane, Silylamine wie Trimethylsilylamin, Silylphosphine wie Trimethylsilylphosphin, Kieselsäuren und deren Ester zusetzt.

7. Verfahren nach den Ansprüchen 1 und 2, dadurch gekennzeichnet, dass man den Ausgangsstoffen als Elektronendonatoren substituierte Schwefelverbindungen, Säuren des Schwefels und deren Ester zusetzt.

8. Verfahren nach den Ansprüchen 1 und 2, dadurch gekennzeichnet, dass man den Ausgangsstoffen als Elektronendonatoren substituierte P-, As-, und Sb-Verbindungen zusetzt.

9. Verfahren nach Anspruch 8, dadurch gekennzeichnet, dass man als Elektronendonatoren die Säuren dieser Elemente und deren Ester, ebenso Alkyl-, Aryl- und Alkoxylverbindungen dieser Elemente zusetzt.

**Claims**

1. A process for the preparation of olefins from methanol and/or dimethyl ether by conversion in the presence of a zeolite catalyst at elevated temperature, wherein the reaction is carried out with the addition of an electron donor in an amount of from 1 to 300 ppm.

2. A process as claimed in Claim 1, wherein the reaction is carried out in the presence of a borosilicate zeolite.

3. A process as claimed in Claims 1 and 2, wherein a substituted hydrocarbon is added to the starting materials as an electron donor.

4. A process as claimed in Claim 3, wherein an organic acid or amine is added to the starting materials as an electron donor.

5. A process as claimed in Claims 1 and 2, wherein a substituted silane is added to the starting materials as an electron donor.

6. A process as claimed in Claims 1 and 2, wherein a suitable silicon-containing compound, such as a siloxane, disiloxane, trisiloxane, silylamine, e.g. trimethylsilylamine, silylphosphine, e.g. trimethylsilylphosphine, or silicic acid or an ester thereof, is added to the starting materials as an electron donor.

7. A process as claimed in Claims 1 and 2, wherein a substituted sulfur compound or an acid of sulfur or ester thereof is added to the starting materials as an electron donor.

8. A process as claimed in Claims 1 and 2, wherein a substituted P, As or Sb compound is added to the starting materials as an electron donor.

9. A process as claimed in Claim 8, wherein an acid of one of these elements or an ester thereof, or an alkyl, aryl or alkoxy compound of one of these elements, is added to the starting materials as an electron donor.

**Revendications**

1. Procédé de préparation d'oléfines à partir du méthanol et/ou de l'éther diméthylique par transformation en présence de catalyseurs du type zéolites à température élevée, caractérisé en ce que l'on effectue la réaction avec adjonction de donneurs d'électrons en quantités de 1 à 300 ppm.

2. Procédé selon la revendication 1, caractérisé en ce que l'on effectue la réaction en présence de zéolites du type borosilicate.

3. Procédé selon les revendications 1 et 2, caractérisé en ce que les donneurs d'électrons ajoutés aux matières premières sont des hydrocarbures substitués.

4. Procédé selon la revendication 3, caractérisé en ce que les donneurs d'électrons ajoutés aux matières premières sont des acides et des amines organiques.

5. Procédé selon les revendications 1 et 2, caractérisé en ce que les donneurs d'électrons ajoutés aux matières premières sont des silanes substitués.

6. Procédé selon les revendications 1 et 2, caractérisé en ce que l'on ajoute aux matières premières, en tant que donneurs d'électrons, des composés appropriés du silicium, tels que des siloxanes, des disiloxanes, des trisiloxanes, des silylamines telles que la triméthylsilylamine, des silylphosphines telles que la triméthylsilylphosphine, des acides siliciques et leurs esters.

7. Procédé selon les revendications 1 et 2, caractérisé en ce que l'on ajoute aux matières premières, en tant que donneurs d'électrons, des composés substitués du soufre, des acides du soufre et leurs esters.

8. Procédé selon les revendications 1 et 2, caractérisé en ce que l'on ajoute aux matières premières, en tant que donneurs d'électrons, des composés substitués de P, As et Sb.

9. Procédé selon la revendication 8, caractérisé en ce que l'on ajoute, en tant que donneurs d'électrons, les acides de ces éléments et leurs esters et, également, les dérivés alkylés, arylés et alcoxylés de ces éléments.